# EUROPEAN PATENT SPECIFICATION

(11) **EP 0 717 625 B1**
(45) Date of publication and mention of the grant of the patent: **12.06.2002**
(21) Application number: 94926719.9
(22) Date of filing: 05.09.1994
(51) Int. Cl.: A61K 31/55, A61K 31/275, A61K 31/44

(54) **A METHOD OF TREATING LIVER DISEASE AND LIKE INDICATIONS WITH VASODILATING AGENTS**
VERFAHREN ZUR BEHANDLUNG VON LEBERERKRANKUNGEN UND ÄHNLICHEN INDIKATIONEN MIT VASODILATIERENDEN WIRKSTOFFEN
METHODE DE TRAITEMENT DE MALADIE HEPATIQUE ET D'INDICATIONS ANALOGUES AU MOYEN D'AGENTS VASODILATATEURS

(30) Priority: 08.09.1993 AU PM110493
(43) Date of publication of application: 26.06.1996
(73) Proprietor: Pharmacy and Therapeutic Advisory Consultancy Ltd., London WC1X 8QT (GB)
(72) Inventor: McCLEAN, Allan Joseph, South Melbourne, VIC 3205 (AU)
(74) Representative: Spall, Christopher John
(86) International application number: AU9400525
(87) International publication number: WO9507080

(56) References cited:
- WO-A-92/04008
- AU-A- 1 632 692
- MARTEAU ET AL.: "Effect of Vasodilators on Hepatic Microcirculation: A Study of the Inhibition of Norepinephrine-Induced Vasoconstriction in the isolated Perfused Rat Liver" HEPATOLOGY, vol. 8, no. 2, 1988, pages 228-231, XP000673345
- MARTEAU ET AL.: "Effect of Vasodilators on Hepatic Microcirculation in Cirrhosis: A Study in the Isolated Perfused Rat Liver." HEPATOLOGY, vol. 9, no. 6, 1989, pages 820-823, XP000673346
- HIERLINGER: "Chronic Verapamil administration improves liver function and diffusional exchange in a rat model of liver cirrhosis" J. HEPATOL., no. suppl. 2, 1985, page S253 XP002030787
- ROMANO ET AL.: "Alterazione degli enzimi epatici in corso di terapia con Diltiazem" G. ITAL. CARDIOL., vol. 17, no. 2, 1987, pages 149-150, XP000673314
- NAVASA ET AL.: "Effects of Verapamil on Hepatic and Systemic Hemodynamics and Liver Function in Patients with Cirrhosis and Portal Hypertension." HEPATOLOGY, vol. 8, no. 4, 1988, pages 850-854, XP000673312
- MERKEL: "Lack of effec of verapamil and isosorbide dinitrate on the hepatic clearance of indocyanine green in cirrhosis." BR. J. CLIN. PHARMACOL., vol. 30, no. 2, 1990, pages 221-228, XP000673273
- GASIC ET AL.: "Comparative effects of verapamil, thiapamil, ditiazem and nifedipine on systemic and splanchnic hemodynamics in man." INT. J. CLIN. PHARMACOL. THER. TOXICOL., vol. 25, no. 9, 1987, pages 498-503, XP000673344
- MACMATHUNA ET AL.: "Vasopressin-nifedipine: a favourable Haemodynamic interaction in cirrhosis and portal hypertension." EUR. J. GASTROENTEROL. HEPATOL., vol. 5, no. 10, 1993, pages 853-857, XP000673271
- PATENT ABSTRACTS OF JAPAN; & JP,A,5 059 028 (OSHIDA), 9 March 1993 (09.03.93).
- PATENT ABSTRACTS OF JAPAN; & JP,A,56 068 619 (YAMANOUCHI SEIYAKU K K), 9 June 1981 (09.06.81).
- THE AMERICAN JOURNAL OF SURGERY, Volume 165, 1993, STEIN, H.J. et al., "Effect of Verapamil on Hepatic Ischemia/Reperfusion Injury", pages 96-99.
- BIOCHEMICAL PHARMACOLOGY, Volume 44, No. 11, 1992, LIANG, D et al., "Protective Effects of the Calcium Antagonists Dilitiazem and TA3090 Against Hepatic Injury Due to Hypoxia", pages 2207-2211.
- BIOCHEMICAL PHARMACOLOGY, Volume 43, No. 4, 1992, LE COUTEUR, D. et al., "Aging and the Response of the Isolated Perfused Rat Liver to Vasoactive Drugs", pages 913-915.
- JOURNAL OF CARDIOVASCULAR PHARMACOLOGY, Volume 20, 1992,, OGAWA, N. et al., "Comparison of KRN2391 with Nicorandil and Nifedipine on Canine Coronary Blood Flow: Antagonism by Glibenclamide", pages 11-17.
- JOURNAL OF SURGICAL RESEARCH, Volume 50, 1991, CHENG, S. et al., "Verapamil Improves Rat Hepatic Preservation with UW Solution", pages 560-564.
- CLINICAL PHARMACOLOGY AND THERAPEUTICS (ST. LOUIS), Volume 50, No. 4, 1991, REISS, W G et al., "The Effect of oral Nifedipine on Hepatic Blood Flow in Humans", pages 379-384.
- PFLUGERS ARCH: EUROPEAN JOURNAL OF PHYSIOLOGY (BERLIN), Volume 415, No. 6, 1990, HELLER, J et al., "The Effect of Two Different Calcium Antagonists on the Glomerular Haemodynamics in the Dog", pages 751-755.
- EUROPEAN JOURNAL OF PHARMACOLOGY, Volume 146, 1988, BUXTON, D B: "Potentiation of the Glycogenolytic and Haemodynamic Actions of Adenosine in the Perfused Rat Liver by Verapamil", pages 121-127.
- JOURNAL OF CLINICAL INVESTIGATION, Volume 78, No. 2, 1986, REICHEN, J et al., "Verapamil Favorably Influences Hepatic Microvascular Exchange and Function in Rats with Cirrhosis of the Liver", pages 448-455.
- BRITISH JOURNAL OF CLINICAL PHARMACOLOGY, Volume 20, No. 2, 1985, MEREDITH et al., "Verapamil Pharmacokinetics and Apparent Hepatic and Renal Blood flow", pages 101-106.
- EUROPEAN JOURNAL OF CLINICAL PHARMACOLOGY, Volume 24, No. 1, 1983, JOHNSSON et al., "Haemodynamic Effects of a New Vasodilator Drug, Felodipine, in Healthy Subjects", pages 49-53
- Rote Liste 1991, 26;105

## Description

The present invention relates to a method of producing a pharmaceutical composition for use in the treatment of liver disease.

Diltiazem is the generic name given to the active component of a composition that is primarily used for the treatment of heart disease. Specifically it is known as 3-acetoxy-5-(2-(dimethylaminoethyl)-2,3-dihydro-2-(4- methoxy phenyl)-1,5-benzothiazepine-4)5H-one. This compound is the active ingredient in the heart treatment drug Cardizem. Cardizem has particular efficacy in the treatment of ischaemic heart disease including angina pectoris and hypertension.

Diltiazem is a member of a broad class of benzothiazepine derivatives that are the subject of Australian patent 426146. The class of compounds are referred to in that specification as having particular utility as anti-depressants, tranquillisers and coronary vasodilators.

Diltiazem primarily acts as a calcium channel antagonist (a calcium blocker); calcium being involved in several biological process in the human body including vasoconstriction and vasodilation. Calcium blockers interfere with the transport of calcium through the cell membrane, thus reducing the contraction of vascular smooth muscle and causing the arteries to dilate. The discovery of calcium blockers constituted a major advance in cardiovascular treatment. Diltiazem contributed significantly to this advance. Generally, during cardiovascular treatment using Diltiazem, a patient in need thereof is administered the drug in doses of from 180 mg to 360 mg per day.

The liver is a large gland situated in the upper part of the abdomen on the right side. Its domed upper surface fits closely against the inferior surface of the right diaphragm. It has a double blood supply from the hepatic artery (oxygenated arterial blood) and the portal vein (deoxygenated venous blood carrying substances absorbed from the stomach, small intestine and large intestine). It comprises thousands of minute lobules (lobuli hepatis), the functional units of the liver. Its manifold functions include the storage and filtration of blood, the secretion of bile, the excretion of bilirubin and other substances formed elsewhere in the body, and numerous metabolic functions, including the conversion of sugars into glycogen, which it stores. It is essential to life and accordingly liver disfunction is debilitating and life threatening.

Prior art treatments of liver disease have included use of a number of drugs. For example, choline has been administered as an adjunct to the dietary treatment of fatty acid infiltration and early cirrhosis of the liver. Methionine has a lipotropic action similar to choline. It has also been used as an adjunct in the treatment of liver diseases in patients unable to take an adequate diet, though there is evidence that in cases of severe liver damage large doses of methionine may aggravate the toxaemia. Litrison is a composition of methionine, choline, vitamins of the B complex and Vitamin E. It has been used for the treatment of hepatic parenchymal degenerative changes and to maintain the function of the liver. Neurogem is a composition of high potency essential Vitamin B-complex and Vitamin C which has been used for supplementary of maintenance therapy. Finally, Ripason is a protein-free total extract from livers of healthy animals. It has been used to treat chronic hepatitis, cirrhosis, medicamentous liver damage and liver parenchyma disorders.

The treatment of liver disease, however, has been an ongoing difficulty in the prior art and none of the drugs used have proved to be particularly effective. In particular, none of these agents reverses the relative hypoxia, or oxygen lack, which appears to contribute to the pathology and progression of chronic liver disease. Accordingly, liver disease continues to be a life-threatening disease and ultimately may require surgery or even transplants in some cases.

Accordingly, it is an object of the present invention to overcome, or at least alleviate, one of more of the difficulties or deficiencies related to the prior art.

Accordingly, the present invention provides a method of producing a pharmaceutical composition for use in the treatment of liver disease according to the pre-characterising portion of claim 1, characterised in that the vasodilating agent is present in an amount less than that required to produce a significant effect on the heart or peripheral circulation whereby said vasodilating agent selectively increases the supply of oxygenated blood to the liver by increasing hepatic arterial inflow.

The vasodilating agent may include a calcium blocker, e.g., a thiazepine derivative, preferably a benzothiazepine derivative, nifedipine, felodipine or verapamil. Other vasodilators may be used indirectly.

The method may be utilised in the treatment of various diseases of the liver such as cirrhosis of a liver, toxic and medicamentary liver damage or liver parenchymic disorders and related diseases such as hepatitis including chronic active hepatitis.

The method may be directional in that significantly lower doses may be used then are normally administered in the treatment of heart disease or like indications.

Whilst we do not wish to be restricted by theory, it is believed that the class of vasodilating agents known as calcium blockers are effective in the treatment of liver disease as they are selectively able to increase the oxygen content to the liver. In particular, it is believed that calcium blockers are effective in the treatment of liver disease as they are selectively able to dilate the hepatic artery. An increase in oxygen level may alleviate the progress of liver disease, since liver performance generally increases with an increase in the oxygen concentration. Common liver diseases, such as chronic hepatitis or cirrhosis of the liver, share as a pathological feature a low concentration of oxygen in the liver.

The vasodilating agent may be a compound of the formula: 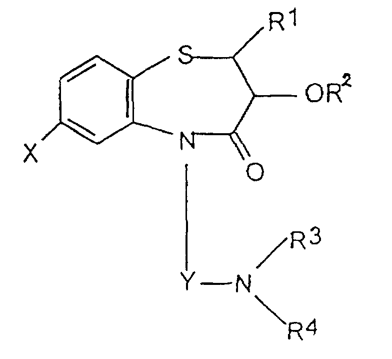 wherein R¹ is a phenyl group substituted or not with 1 to 3 lower alkyl groups, lower alkoxy groups or halogen atoms, R² is a hydrogen atom or a lower alkanoyl group, R³ and R⁴ are each a lower alkyl group and may be the same or different, X is a hydrogen atom or a halogen atom and Y is an alkylene group of 2 or 3 carbon atoms, or its non-toxic acid-addition salt.

Preferably R¹ is 4-lower alkoxyphenyl, R² is lower alkanoyl, R³ and R⁴ are each lower alkyl, X is hydrogen and Y is ethylene. More preferably R¹ is 4-methoxyphenyl, R² is acetyl and R³ and R⁴ are each methyl. Still more preferably, the benzothiazepine derivative is 3-acetoxy-5-(2-(dimethylaminoethyl)-2,3- dihydro-2-(4-methoxy phenyl)-1,5-benzothiazepine-4)5H-one.

The benzothiazepine derivative may be converted into its acid-addition salts by treatment with an organic or inorganic acid (e.g. acetic acid, oxalic acid, malonic acid, tartaric acid, malic acid, citric acid, lactic acid, gluconic acid, aspartic acid, hydrochloric acid, hydrobromic acid, sulfuric acid, nitric acid, perchloric acid, etc.) in a suitable solvent (e.g. water, methanol, ethanol, etc.). It has been found that the use of such benzothiazepine derivatives is effective in increasing the hepatic arterial blood flow to the liver. Such benzothiazepine derivatives may be effective in the treatment of liver disease in significantly lower doses than is normally administered in the treatment of heart diseases. Significantly lower mean doses which will have no significant effect on heart or peripheral circulation.

The pharmaceutical composition of the present invention may be used in a method of treating liver disease which method includes administering to a patient in need thereof a low dose, e.g. of approximately 30 to 100 mg per day preferably 30 to 60 mg/day of diltiazem or its non-toxic acid-addition salt. Experimental studies in mice to date have indicated effective doses of approximately 1.0 to 2.0 mg/kg/day. However, it is common for human doses to be lower than for animals including rodants.

Further disclosed herein is a pharmaceutical composition suitable for the treatment of liver disease and like indications which composition includes a therapeutically or prophylactically effective amount of a vasodilating agent which selectively increases the supply of oxygenated blood to the liver and a pharmaceutically acceptable diluent or carrier therefor.

The vasodilating agent may include a calcium blocker, e.g. a thiazepine derivative, preferably a benzothiazepine derivative, nifedipine, felodipine or verapamil. Other vasodilators may be used indirectly.

The pharmaceutical composition may be utilised in the treatment of various diseases of the liver such as cirrhosis of a liver, toxic and medicamentary liver damage or liver parenchymic disorders and related diseases such as hepatitis including chromic active hepatitis.

The composition may include a therapeutically or prophylactically effective amount of a benzothiazepine derivative of the formula: 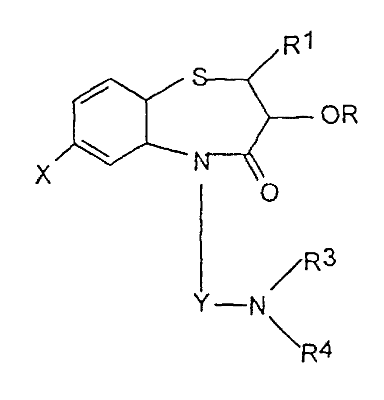 wherein R¹ is a phenyl group substituted or not with 1 to 3 lower alkyl groups, lower alkoxy groups or halogen atoms, R² is a hydrogen atom or a lower alkanoyl group, R³ and R⁴ are each a lower alkyl group and may be the same or different, X is a hydrogen atom or a halogen atom and Y is an alkylene group of 2 or 3 carbon atoms, or its non-toxic acid-addition salt; and
a pharmaceutically acceptable diluent or carrier therefor.

Preferably R¹ is 4-lower alkoxyphenyl, R² is lower alkanoyl, R³ and R⁴ are each lower alkyl, X is hydrogen and Y is ethylene More preferably R¹ is 4-methoxyphenyl, R² is acetyl and R³ and R⁴ are each methyl. Still more preferably, the benzothiazepine derivative is 3-acetoxy-5-(2-(dimethylaminoethyl)-2,3- dihydro-2-(4-methoxy phenyl)-1,5-benzothiazepine-4)5H-one.

Further disclosed is a pharmaceutical composition suitable for the treatment of liver disease and like indications which composition includes approximately 30 to 60 mg of diltiazem or its non-toxic acid-addition salt, and a pharmaceutically acceptable diluent or carrier therefor.

The pharmaceutically acceptable diluent or carrier may be of any suitable type. The pharmaceutically acceptable diluent or carrier may be a pharmaceutical organic or inorganic carrier material suitable for enteral, parenteral or transdermal applications.

Preferably the composition is formulated so as to allow suitable administration to the patient. Such administration may be by any suitable means such as oral, subcutaneous, intravenous or transcutaneous. Preferably administration is by oral route as the active ingredient is able to reach the liver directly, that is through the portal vein.

Oral administration by the use of tablets, capsules, powders or in liquid form such as suspensions, solutions, emulsions or syrups is particularly advantageous. When formed into tablets, conventional excipients (e.g. sodium citrate, lactose, microcrystalline cellulose, starch, etc.), lubricating agents (e.g. anhydrous silicic acid, hydrozed castor oil, magnesium stearate, sodium lauryl sulfate, talc, etc.) and binding agents (e.g. starch pasto, glucose, lactose, gum acacia, gelatin, mannitol, magnesium trisilicate, talc, etc.) can be used.

When administered as liquids, conventional liquid carriers can be employed. In the case of solid preparations, each unit dosage form of the active ingredient can contain from about 5 to about 95% of the same by weight of the entire composition with the remainder comprising conventional pharmaceutical carriers. When the therapeutic agent is used as aqueous solution, i.e. injection, the solution may contain about 0 05 to about 0.5% of the same by weight of the entire solution.

Preferably the composition may be of the sustained release type, for example to allow for a once-daily administration. The sustained release composition may be suitable for oral or transdermal administration. A suitable slow release formulation may be achieved for example when the active ingredient is bound to a suitable polymer. A once daily composition is able to supply sufficient quantity of active ingredient to the patient.

The present invention will now be more fully described with reference to the accompanying examples. It should be understood, however, that the description following is illustrative only and should not be taken in any way as a restriction on the generality of the invention described above.

### EXAMPLE 1

### AIM:

A pilot study was undertaken to examine the effect on hepatic artery and mesenteric artery flow in anaesthetised dogs when exposed to cumulative doses of diltiazem.

### METHOD:

### Preparation:

Greyhounds were used in this pilot study. All dogs were present in the animal house for < 1 week prior to surgery, and all were deemed clinically sound. Dogs were given 15 minutes of exercise prior to arriving at the theatre. On arrival, they were clipped on the abdomen, forelimbs and hindquarters, and anaesthesia was induced with sodium pentobarbitone (Nembutal or InjectionT) given intravenously to effect. Subjects were intubated and connected to a respirator. Table heating was used to maintain body temperature. An initial infusion of 1 litre of Hartmann's solution was given throughout the surgical procedure, with bicarbonate being administered as required according to blood gas estimation.

The abdomen was opened, and the gastro-duodenal branch of the common hepatic artery was located and ligated. Electromagnetic flow probes were placed on the common hepatic artery and the anterior mesenteric artery. A branch of the splenic vein was exposed and a catheter introduced and advanced into the portal vein. A catheter was also placed in the left hepatic vein using a purse string technique. An indwelling catheter was placed in a branch of the mesenteric vein, in close proximity to another catheter placed in the lumen of the jejunum. The abdomen was then closed and a catheter introduced into the femoral artery.

The subject was then covered with drapes, and the dogs circulation and temperature allowed to stabilise prior to the commencement of the experimental stage.

At the end of the study, the dogs were euthanased with sodium pentobarbitone.

### Experimental Procedure:

Theophylline was used infused as a marker of liver extraction. A bolus was given (over 15 minutes) at a rate of 3.42 mg/min, then an infusion into the mesenteric vein at a rate of 11 mg/min. After 90 minutes stabilisation, the first dose of diltiazem was given (0.25 mg/kg) into a jejunal lumen. Time was allowed for any changes in blood flow before the next dose was given. Effects on flow reached a plateau by 20 minutes, when the next dose was given. Cumulative doses were given, i.e. 0.25, 0.5, 1.0, 2.0, 4.0 mg/kg. Blood samples were taken throughout the procedure from the portal vein, posterior hepatic vein and arterial line at 20, 40, 60, 80, 90, 100, 110, 120, 130, 140, 150, 160, 170, 180 and 190 minutes, with zero time being the start of the theophylline infusion.

### RESULTS:

6 dog Studies were performed, as per the summary in Table 1 below.

**Table 1**

| Dog | Preparation | Experimental |
|---|---|---|
| Dog 3/92 | Surgery went well | No flow responses |
| Dog 4/92 | Surgery went well | Excellent flow responses to diltiazem |
| Dog 1/93 | Surgery went well | Flow response to diltiazem |
| Dog 2/93 | Surgery went well | Excellent flow responses to diltiazem |
| Dog 3/93 | Surgery unsuccessful | - |
| Dog 4/93 | Surgery OK | Flow responses to diltiazem |

### STATISTICAL OBSERVATIONS

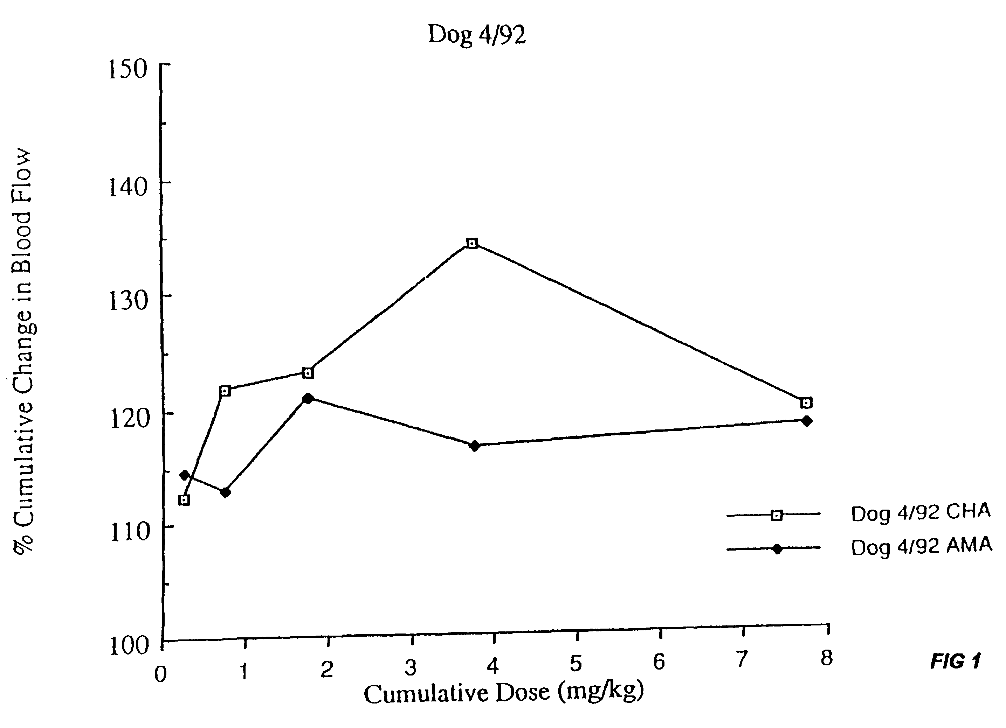

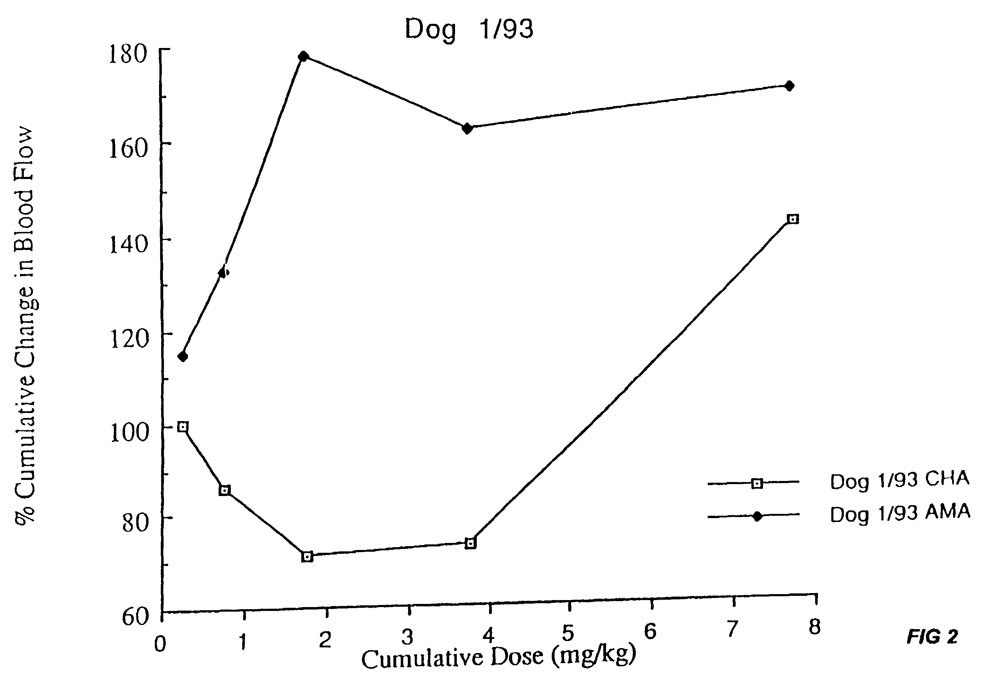

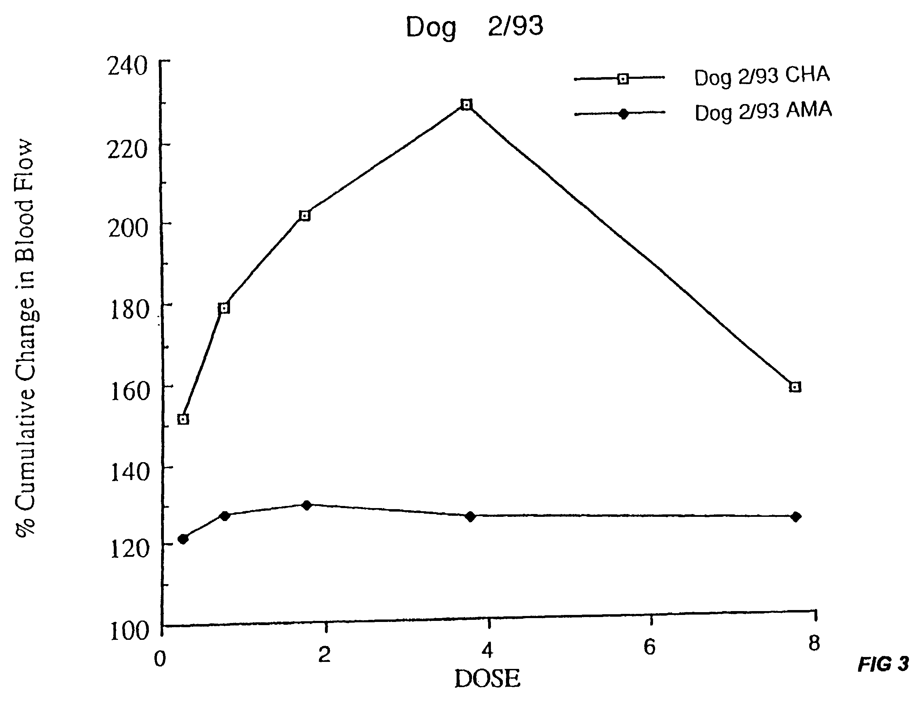

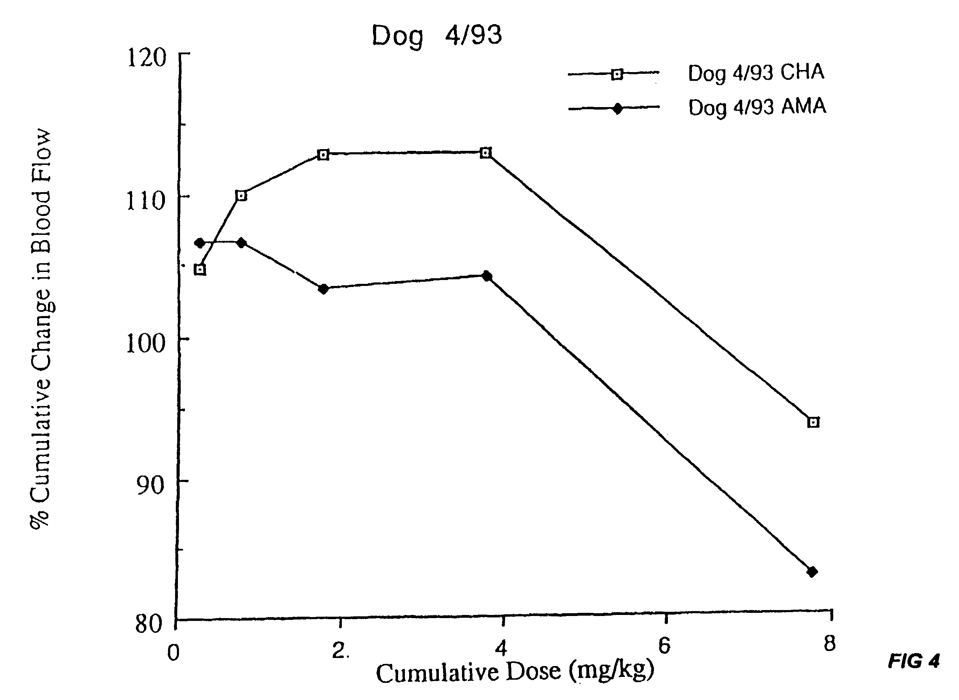

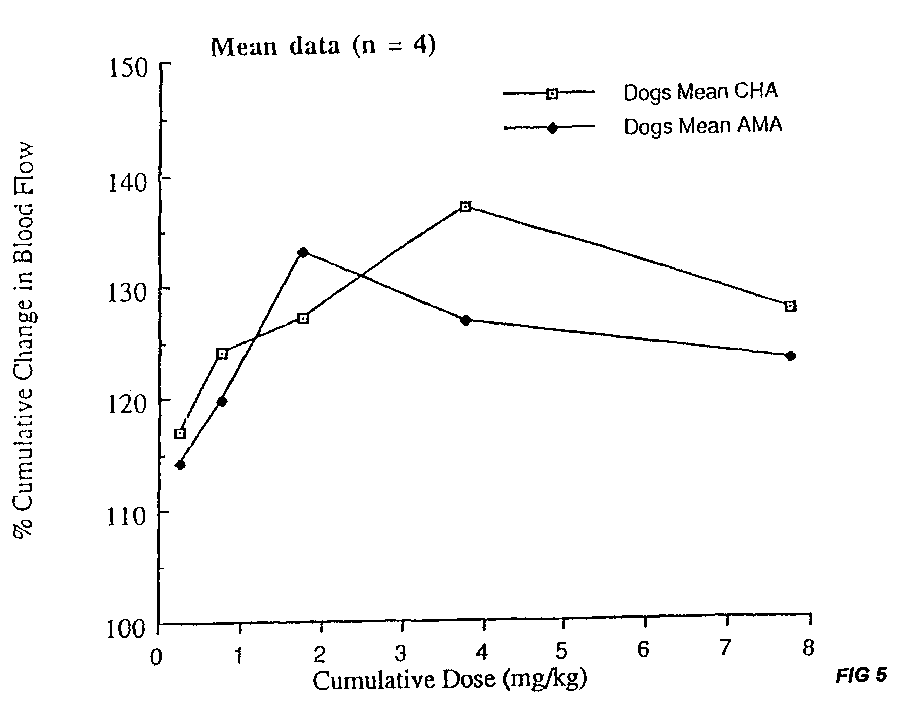

Mean flows were obtained in both the hepatic and mesenteric arteries for 10 to 20 minutes prior to diltiazem being given. This was taken as baseline flows, and all measurements used this as baseline. Maximum flow responses were measured. The results are summarised in the tables 2a and b below and are presented diagrammatically in Figures 1-5.

**Table 2a**

| Common Hepatic Artery | | | | | |
|---|---|---|---|---|---|
| Subject | +%CHA 0.25mg/kg | +%CHA 0.5mg/kg | +%CHA 1.0mg/kg | +%CHA 2.0mg/kg | +%CHA 4.0mg/kg |
| 4/92 | 112.4 | 121.7 | 123.2 | 134.0 | 119.6 |
| 1/93 | 99.9 | 86.0 | 71.2 | 73.2 | 140.7 |
| 2/93 | 151.5 | 178.7 | 201.3 | 227.6 | 156.0 |
| 4/93 | 104.7 | 110.1 | 112.8 | 112.8 | 93.5 |
| MEAN | 117.1±11.7 | 124.1±19.7 | 127.1±27.2 | 136.9±32.8 | 127.5±13.6 |

**Table 2b**

| Anterior Mesenteric Artery | | | | | |
|---|---|---|---|---|---|
| Subject | +%AMA 0.25mg/kg | +%AMA 0.5mg/kg | +%AMA 1.0mg/kg | +%AMA 2.0mg/kg | +%AMA 4.0mg/kg |
| 4/92 | 114.6 | 112.9 | 120.9 | 116.6 | 118.1 |
| 1/93 | 114.9 | 132.4 | 177.8 | 161.5 | 168.3 |
| 2/93 | 121.1 | 127.0 | 129.3 | 125.1 | 123.2 |
| 4/93 | 106.7 | 106.7 | 103.3 | 104.1 | 82.8 |
| MEAN | 114.3±3.0 | 119.8±6.0 | 132.9±15.9 | 126.8±12.3 | 123.1±17.5 |

### EXAMPLE 2

Two sets of experiments were performed. Both were conducted in dogs anaesthetised with barbiturates.

In the first series nitroglycerin was infused into either the portal vein (draining to the liver from the bowel) or to the femoral vein (systemic circulation). When nitroglycerin was given into the portal vein the blood flow through the hepatic artery, (ie. a measure of liver blood flow and oxygenation) increased. By contrast when nitroglycerin was given systemically, hepatic blood flow reduced. It can be concluded that hepatic blood flow and liver oxygenation can both be augmented by drugs, but this cannot be achieved by systemic administration of nitroglycerin.

In the second series, diltiazem was administered by a gastric tube into the stomach - effectively orally. The level of blood flow through the hepatic artery increased by up to 50%, and this occurred at very small doses. Thus, increase in liver perfusion may be achieved by small doses of oral diltiazem and this will have a benefit on the diseased liver.

### EXAMPLE 3

A third set of experiments was then undertaken in rats after the earlier studies in dogs had shown that low doses of diltiazem increased liver blood flow. The aim of the study was to induce liver disease by administration of carbon tetrachloride (CCl₄) and then test the hypothesis that low doses of diltiazem would improve the functional state of the liver.

### METHODS

Male Sprague Dawley rats were used in this study in which liver disease was induced after the method of Proctor and Chatamra (1982). Hepatic enzymes were first induced by addition of sodium phenobarbitone to the drinking water to a concentration of 350mg/100ml. All animals were given the phenobarbitone water for 10 weeks; no other water was available to the animals.

Animals randomised for induction of liver disease received CCl₄ added to maize oil, and administered orally through a stainless steel gavage tube during carbon dioxide stun. The CCl₄ was given for ten weeks as weekly doses commencing after two weeks of enzyme induction with phenobarbitone sodium. The starting dose of CCl₄ was 0.5ml but the dose was then adjusted according to protocol to achieve a weight loss of 6 to 9% over the 3 days after each dose, with weight gain by day 7. Previous studies have shown that over a period of ten weeks, this regimen will produce liver disease with ascites, splenomegaly, reduction of plasma albumin, increase of plasma alanine transaminase, and the histological features of severe liver disease.

For the assessment of the effects of diltiazem, animals were separated into five groups each of 8 rats. Group 1 (normal) received phenobarbitone in the drinking water but no CCl₄ or diltiazem. Group 2 (control) received CCl₄ but no diltiazem. Groups 3, 4 and 5 received respectively 0.5, 1.0 and 2.0 mg/kg per day of diltiazem added to the drinking water.

The animals were weighed daily for the four days after each dose of CCl₄, and sacrificed after 12 weeks, that is, after 10 weeks of CCl₄ +/- diltiazem, or at the equivalent time in normal animals. At autopsy, the weights of the livers and spleens were recorded, the presence of ascites and the coat condition was noted, and blood samples were taken for measurement of albumin, liver enzymes and blood clotting factors.

The between group differences for each variable were examined using analysis of variance.

### RESULTS

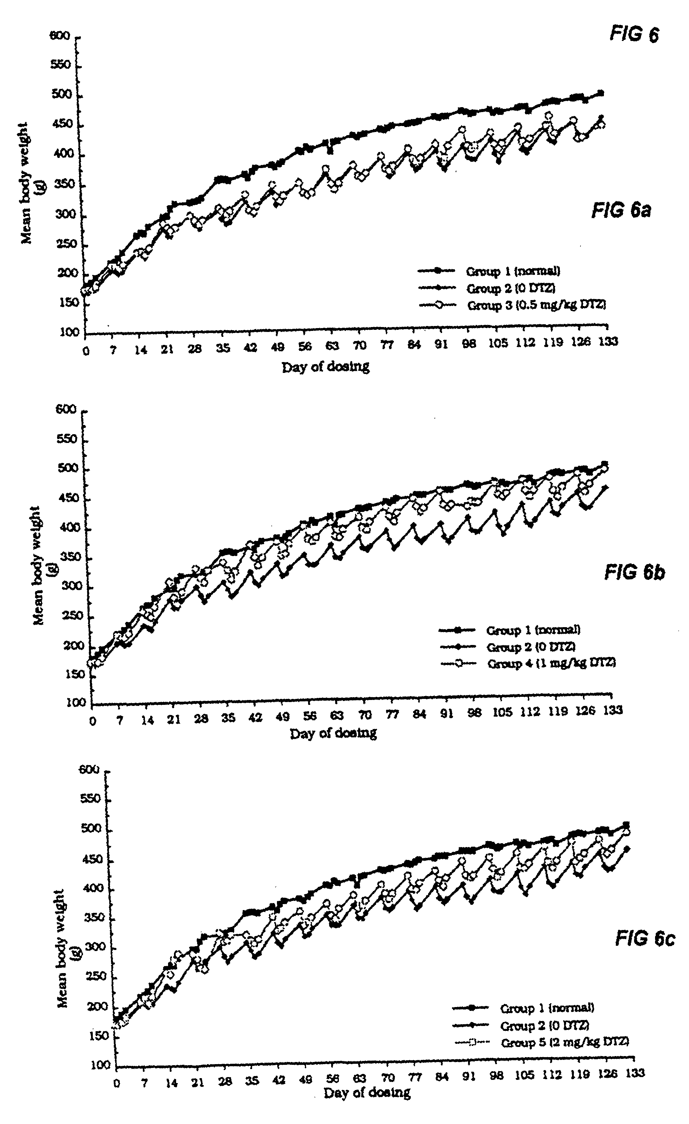

The body weight profiles are shown in Figures 6a, b, c, which show mean rat body weight profiles during DTZ administration, and CC1₄ dosing for induction of cirrhosis in normal, nil DTZ and (6a) 0.5 (6b) 1.0 (6c) 2.0 mg/kg body weight. Group 1 (normal) animals progressively increased in weight from less than 200 grams to about 440 grams body weight over the study period. Group 2 (control) lost weight after each dose of CCl₄, and did not gain as much weight as Group 1 being 50 to 60 grams lighter at the end of the study period.

Treatment with 0.5mg/kg/day of diltiazem (Group 2) appeared to have no significant effect of preventing CCl₄-induced weight loss. By contrast, in Group 3 (treated with 1.0 mg/kg/day of diltiazem), there was a transitory loss in weight after each dose of CCl₄.

However by the end of the study, body weights were not significantly different from normal (Group 1) but were significantly heavier than those of control animals (Group 2; p<0.05). The effects of 2.0mg/kg/day (Group 5) appeared to be less than that of 1.0mg/kg/day.

Autopsy and biochemistry variables are listed in Table 3. In Groups 1 (normal) and 4 (diitiazem, 1.0mg/kg/day) the liver and spleen appeared normal to inspection, and there was not significant ascites. By contrast Group 2 (control) showed evidence of severe liver disease. The macroscopic changes seen in the control group are supported by the reduction of plasma albumin and clotting factors and increase in plasma alanine transaminase compared with levels in the normal group of animals. Diltiazem afforded significant protection against the development of liver disease as evidenced by the protection against loss of body weight and increase in spleen size and this effect appeared to be greatest at the 1.0mg/kg/day dose. Those primary indicators are supported by the increased protection against enzyme release. However protection against enzyme release was slightly better at the 2.0 mg/kg/day dose.

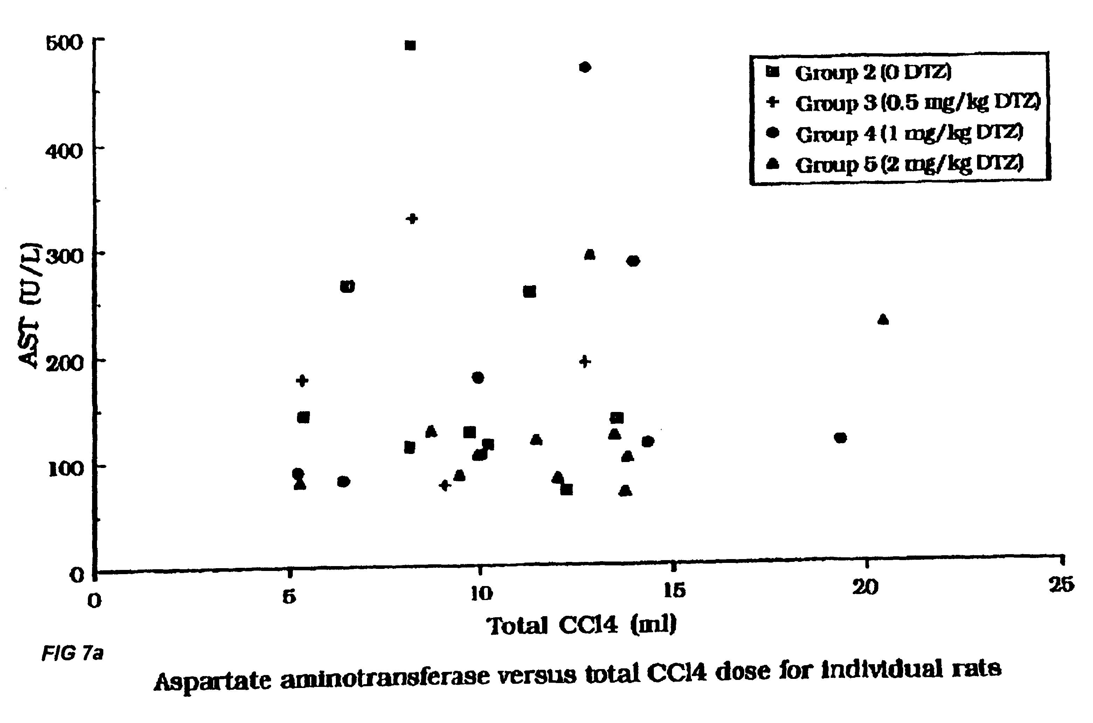

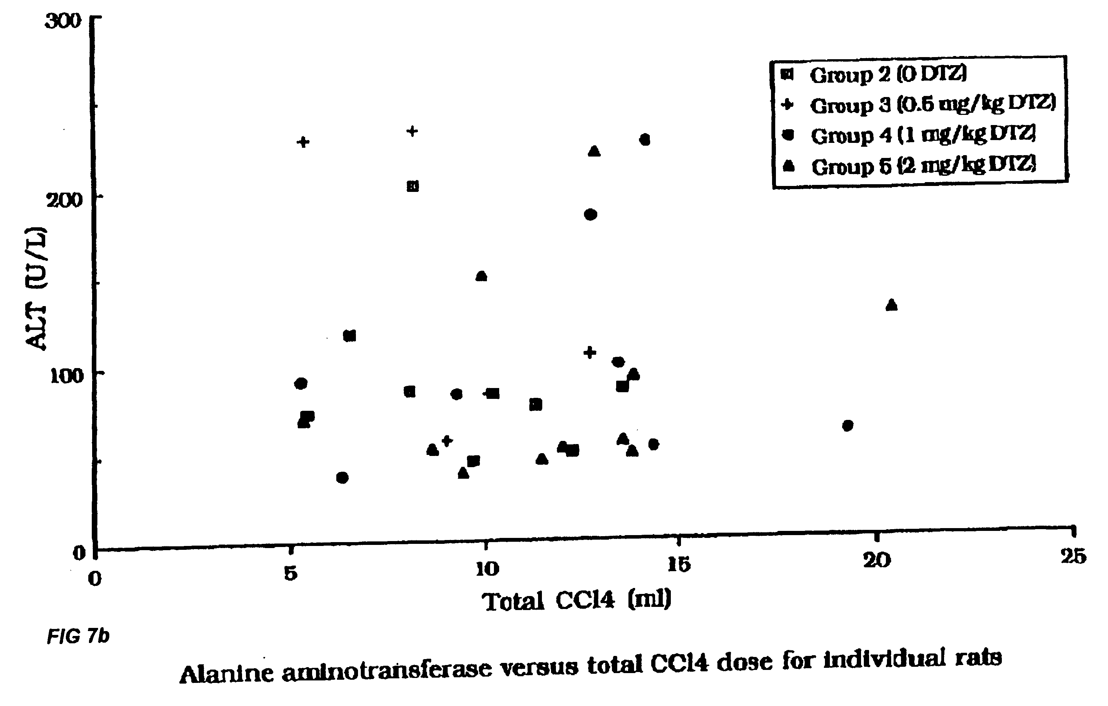

The result reported in Table 3 do, however, somewhat underestimate the protective effects of Diltiazem against liver disease as the Trial protocol means that healthy animals receive more CCl₄ than animals showing signs of liver disease because the weekly dose of CCl₄ was titrated against weight loss. The effect of this is illustrated in Figure 7. Figures 7a and b are respectively plots of AST and ALT Enzyme release vs - Total Body load of CCl₄.

### DISCUSSION AND CONCLUSION

The results of this study in rats show conclusively that low doses of diltiazem significantly prevented the development of liver disease in rats administered with CCl₄. Particularly significant is the observation that the greatest effect of diltiazem appeared with a dose of 1.0mg/kg/day, in respect of body weight and spleen size (an indicator of portal vein congestions), rather than 0.5 or 2.0mg/kg/day. The previous studies in dogs suggest that the mechanism of action is likely to be an increase in blood flow to the liver, and hence increased oxygenation of the liver. These observations in animals should now be tested in human patients with liver disease. These studies strongly suggest that it will be low doses of Diltiazem which will be effective in treating liver disease in man.

Finally, it is to be understood that various other modifications and/or alterations may be made without departing from the spirit of the present invention as outlined herein.

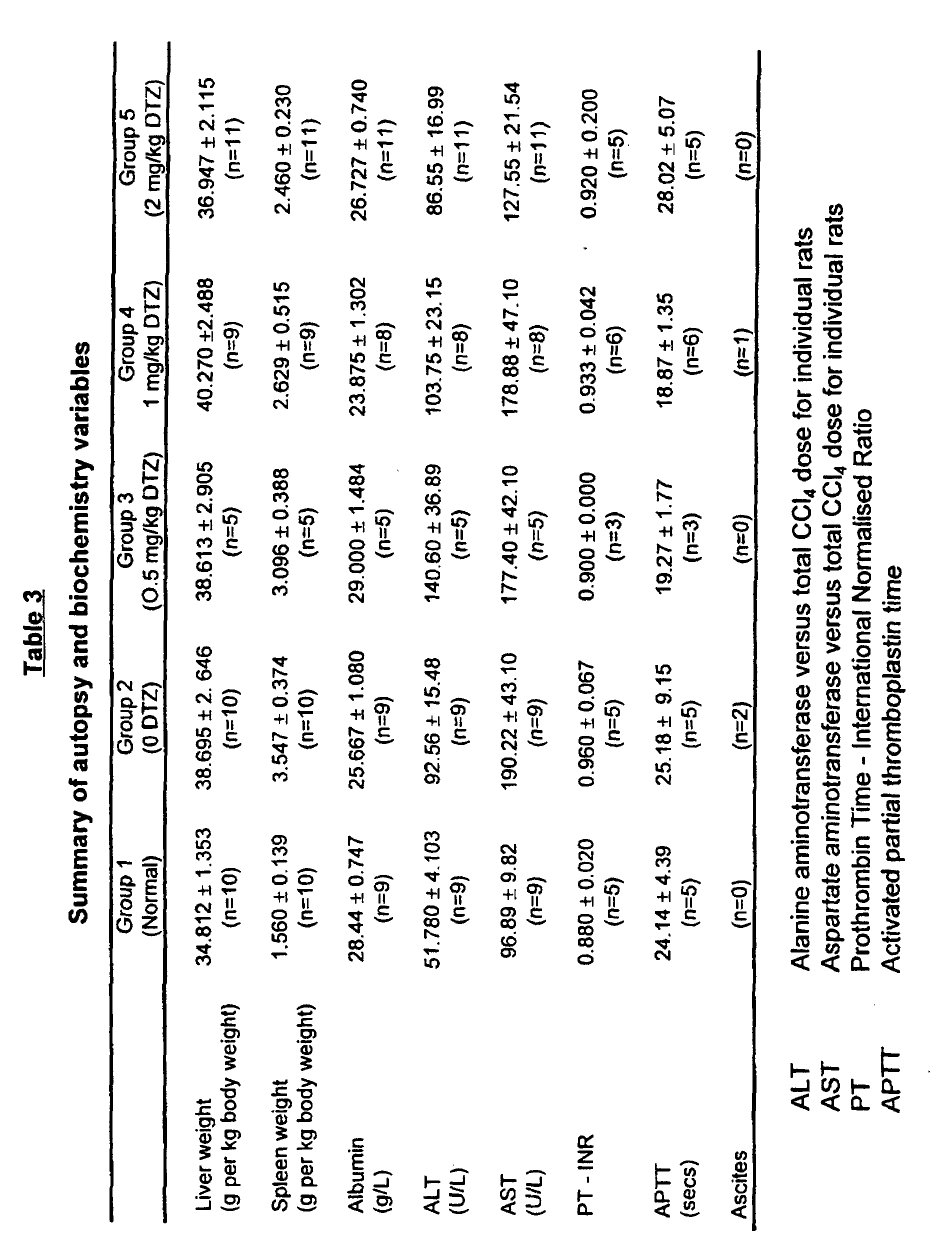

## Claims

1. A method of producing a pharmaceutical composition for use in the treatment of liver disease selected from the group consisting of cirrhosis of the liver, toxic and medicamentary liver damage, a liver - parenchymic disorder or hepatitis, by forming a mixture of a vasodilating agent and a pharmaceutically acceptable diluent or carrier in a form suitable for administration **characterised** that the vasodilating agent is present in an amount less than that required to produce a significant effect on the heart or peripheral circulation whereby said vasodilating agent selectively increases the supply of oxygenated blood to the liver by increasing hepatic arterial inflow.

2. A method according to claim 1 wherein the pharmaceutically acceptable diluent or carrier is in a form suitable for oral administration.

3. A method according to claim 1 wherein the vasodilating agent is a calcium blocker.

4. A method in accordance with claim 1 wherein the vasodilating agent is selected from the group consisting of a thiazepine derivative, nifedipine, felodipine, and verapamil.

5. A method in accordance with claim 4 wherein the vasodilating agent is a compound of the formula: 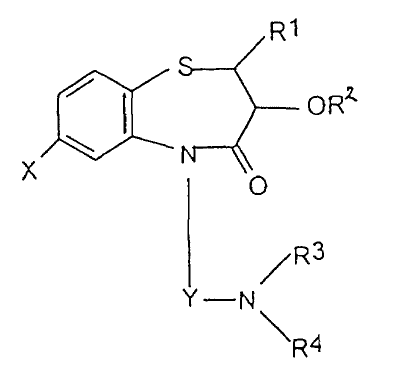 wherein R¹ is a phenyl group substituted or not with 1 to 3 lower alkyl groups, lower alkoxy groups or halogen atoms, R² is a hydrogen atom or a lower alkanoyl group, R³ and R⁴ are each a lower alkyl group and may be the same or different, X is a hydrogen atom or a halogen atom and Y is an alkylene group of 2 or 3 carbon atoms, or its non-toxic acid-addition salt.

6. A method according to claim 5 wherein the vasodilating agent is diltiazem.

7. A method according to any one of the previous claims wherein the vasodilating agent is present in the range of from 30 to 100 mg.

8. A method according to claim 6 wherein the diltiazem is present in the range of 30 to 60 mg.

## Patentansprüche

1. Verfahren zum Herstellen einer pharmazeutischen Zusammensetzung für eine Verwendung bei der Behandlung von Lebererkrankungen, ausgewählt aus der Gruppe bestehend aus Leberzirrhose, toxischer und medikamentenbedingter Leberschädigung, einer Leberparenchymerkrankung oder Hepatitis, durch Bilden einer Mischung eines gefäßerweiternden Mittels und eines pharmazeutisch annehmbaren Verdünnungsmittels oder Trägers in einer für eine Verabreichung geeigneten Form, **dadurch gekennzeichnet, dass** das gefäßerweiternde Mittel in einer geringeren Menge als jener, die erforderlich wäre, um eine signifikante Wirkung auf das Herz oder den peripheren Kreislauf zu erzeugen, vorhanden ist, wodurch das gefäßerweiternde Mittel durch Erhöhen des Leberarterieneinstroms selektiv die Zufuhr von sauerstoffreichem Blut zu der Leber erhöht.

2. Verfahren nach Anspruch 1, wobei das pharmazeutisch annehmbare Verdünnungsmittel oder der pharmazeutisch annehmbare Träger in einer für eine orale Verabreichung geeigneten Form vorliegt.

3. Verfahren nach Anspruch 1, wobei das gefäßerweiternde Mittel ein Calcium-Blocker ist.

4. Verfahren nach Anspruch 1, wobei das gefäßerweiternde Mittel aus der Gruppe bestehend aus einem Thiazepinderivat, Nifedipin, Felodipin und Verapamil ausgewählt ist.

5. Verfahren nach Anspruch 4, wobei das gefäßerweiternde Mittel eine Verbindung der Formel: 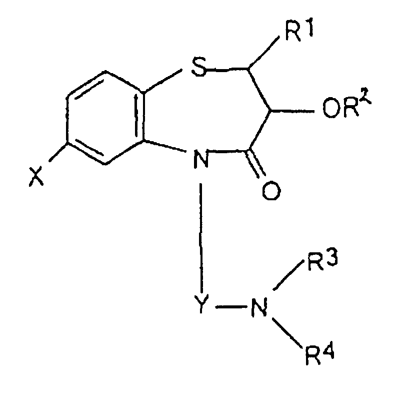 worin R¹ eine gegebenenfalls mit 1 bis 3 Niederalkylgruppen, Niederalkoxygruppen oder Halogenatomen substituierte Phenylgruppe ist, R² ein Wasserstoffatom oder eine niedere Alkanoylgruppe ist, R³ und R⁴ jeweils eine Niederalkylgruppe sind und gleich oder verschieden sein können, X ein Wasserstoffatom oder ein Halogenatom ist und Y eine Alkylengruppe mit 2 oder 3 Kohlenstoffatomen ist, oder deren nicht-toxisches Säureadditionssalz ist.

6. Verfahren nach Anspruch 5, wobei das gefäßerweiternde Mittel Diltiazem ist.

7. Verfahren nach einem der vorangegangenen Ansprüche, wobei das gefäßerweiternde Mittel in dem Bereich von 30 bis 100 mg vorhanden ist.

8. Verfahren nach Anspruch 6, wobei das Diltiazem in dem Bereich von 30 bis 60 mg vorhanden ist.

## Revendications

1. Procédé de production d'une composition pharmaceutique en vue de l'utilisation dans le traitement de maladies hépatiques choisies au sein du groupe comprenant la cirrhose du foie, la lésion hépatique toxique et médicamenteuse, le trouble hépatique parenchymateux ou l'hépatite, par la formation d'un mélange d'agent vasodilatateur et d'un diluant ou véhicule pharmaceutiquement acceptable, sous une forme appropriée à l'administration, **caractérisé en ce que** l'agent vasodilatateur est présent en quantité inférieure à celle requise pour produire un effet significatif sur la circulation cardiaque ou périphérique, grâce à quoi ledit agent vasodilatateur augmente sélectivement l'apport de sang oxygéné au foie en augmentant le flux artériel hépatique entrant.

2. Procédé selon la revendication 1, dans lequel le diluant ou véhicule pharmaceutiquement acceptable est sous une forme appropriée à l'administration orale.

3. Procédé selon la revendication 1, dans lequel l'agent vasodilatateur est un bloquant du calcium.

4. Procédé selon la revendication 1, dans lequel l'agent vasodilatateur est choisi au sein du groupe comprenant un dérivé de thiazépine, la nifépidine, la félodipine et le vérapamil.

5. Procédé selon la revendication 4, dans lequel l'agent vasodilatateur est un composé de formule : 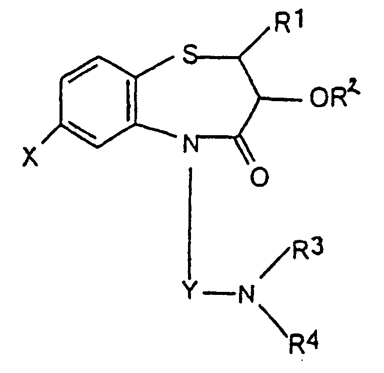 dans laquelle R¹ représente un groupe phényle substitué ou non par 1 à 3 groupes alkyle inférieur, groupes alcoxy inférieur ou atomes d'halogène, R² représente un atome d'hydrogène ou un groupe alcanoyle inférieur, R³ et R⁴ représentent chacun un groupe alkyle inférieur et peuvent être identiques ou différents, X représente un atome d'hydrogène ou un atome d'halogène, et Y représente un groupe alkylène à 2 ou 3 atomes de carbone, ou son sel d'addition d'acide, non-toxique.

6. Procédé selon la revendication 5, dans lequel l'agent vasodilatateur est le diltiazem.

7. Procédé selon l'une quelconque des revendications précédentes, dans lequel l'agent vasodilatateur est présent à raison de 30 à 100 mg.

8. Procédé selon la revendication 6, dans lequel le diltiazem est présent à raison de 30 à 60 mg.
